Europäisches Patentamt

European Patent Office

Office européen des brevets

(19)

(11) Publication number: **0 305 687 B1**

(12) **EUROPEAN PATENT SPECIFICATION**

(45) Date of publication of patent specification: **01.04.92**  (51) Int. Cl.5: **B01D 61/00**, //A61M1/18

(21) Application number: **88111004.3**

(22) Date of filing: **09.07.88**

(54) **A diffusion and/or filtration apparatus and a method of manufacturing said apparatus.**

(30) Priority: **31.08.87 SE 8703368**

(43) Date of publication of application:
**08.03.89 Bulletin 89/10**

(45) Publication of the grant of the patent:
**01.04.92 Bulletin 92/14**

(84) Designated Contracting States:
**BE CH DE ES FR GB IT LI NL**

(56) References cited:
**EP-A- 0 046 889      FR-A- 2 344 262**
**FR-A- 2 456 536      GB-A- 1 175 689**
**GB-A- 2 053 725      SE-B- 454 847**
**US-A- 4 334 993**

**PATENT ABSTRACTS OF JAPAN, vol. 10, no. 339 (C-385)[2395], 15th November 1986; & JP-A-61 141 904 (DAICEL CHEM. IND. LTD) 28-06-1986**

**PATENT ABSTRACTS OF JAPAN, vol. 8, no. 255 (C-253)(1692), 21st November 1984; & JP-A-59 136 104 (ASAHI KASEI KOGYO K.K.) 04-08-1984**

(73) Proprietor: **Gambro Dialysatoren GmbH & Co. KG**
**Postfach 1323**
**W-7450 Hechingen(DE)**

(72) Inventor: **Raff, Manfred**
**Weiherstrasse 21**
**W-7457 Bisingen/Thanheim(DE)**
Inventor: **Spranger, Kurt**
**Obere Strasse 43**
**W-7403 Ammerbuch-Entringen(DE)**

(74) Representative: **Boberg, Nils Gunnar Erik**
**Gambro AB Patent Department Box 10101**
**S-220 10 Lund(SE)**

EP 0 305 687 B1

# Description

## TECHNICAL FIELD

The present invention relates to a diffusion and/or filtration apparatus, including a housing consisting of a cylindrical open-ended main part closed by two end caps and being provided with an inlet and an outlet for a first fluid and at least one outlet for a second fluid, said first fluid being adapted to flow through the fibres of a bundle of semipermeable hollow fibers arranged between two end walls within the housing and said second fluid being adapted to be removed from the space outside the fibers through said at least one outlet for the second fluid, the ends of the hollow fibers being moulded into said end walls and opened by a cutting.

The invention relates furthermore to a method of manufacturing the above defined apparatus.

Devices of the above kind are used for e g different kinds of medical treatment such as hemodialysis, hemofiltration, plasmapheresis and immunotherapy. Other fields of use are for instance dialysis in general and filtration in general, for example in connection with cleaning or desalination of sea water.

## TECHNICAL STANDPOINT

In connection with the production of devices of the above kind each end of the housing with a bundle of fibres placed therein is normally enclosed in a casting mould to which the material for the adjacent end wall is fed in a liquid condition penetrating into and around the fibers. The penetration is so controlled by the casting that the penetration into the fibers is less than around the fibers, making it possible to open the ends of the fibers by making a cut between the surfaces restricting the two different penetration depths, respectively.

This control may be made in different ways. Preferably it is, however, made in accordance with the method described and claimed in EP-B-0 165 478. The wall material may be fed into the device as described in the above European patent or through the other two normally existing side inlets/outlets as described in US-A-4 227 295 or US-A-4 329 229. In both cases the wall material has normally up to today been firmly attached to the inside wall of the housing.

During the curing of the wall material stresses have, however, been built into the material, which sometimes later have been released in connection with the further treatment of the device, providing unacceptable cracks in the wall. Such cracks may for instance be created in connection with the opening of the fiber ends or the flushing or sterilization of the ready made apparatus.

An attempt to solve the above problem is described and claimed in US-A-4 334 993. The present invention relates to a different way to solve the same problems, providing at the same time additional advantages as described below.

## DESCRIPTION OF THE INVENTION

The present invention relates to a diffusion and/or filtration apparatus of the kind defined above, characterized in that the attachment between the moulded end walls and the housing is reduced or eliminated by a ring having a low adhesive capacity as regards the material of the end walls and in that said end walls are supported in the longitudinal direction of the housing.

Thanks to the above defined construction the risk of cracks is essentially reduced. It may therefore even be heat-sterilized in dry condition, if you choose suitable materials for the details involved. An example of how such a heat sterilization may be carried through is described more in detail in US-A-4 609 728. From the following description it will be seen that at the same time are no problems to provide an effective seal between the two fluids involved.

Said longitudinal support of the end walls is preferably provided by a flange arranged on the ring. Such a flange may for instance be arranged on the inside of the ring penetrating into the perifery of the end walls. Such a construction may also be used for supporting the fiber bundle during its treatment before the casting of the end walls.

The ring may be supported in the longitudinal direction by the housing, preferably by being arranged in a groove in the inside wall of the housing. Instead of the above mentioned flange the ring may have another dimensionally restricted part providing a support for the bundle of hollow fibers especially before the moulding of the end walls.

Said ring is preferably extended outside the housing in order to serve as part of the mould in connection with the moulding of the end walls and is being cut together with the fibers in connection with the opening of the fiber ends.

By a preferred embodiment of the apparatus according to the invention a second ring may be arranged at the outer end surfaces of the end walls providing a groove in each of said end surfaces, said ring having also a low adhesive capacity as regards the material of the end walls.

Said support in the longitudinal direction of the housing may be provided by said housing in that the end walls are provided within two expanded parts of the housing and in that the support in the longitudinal direction is provided by tongues which are integral with the housing and partly moulded into the end walls. By such a construction the ring may be given a very simple form.

The invention relates also to a method of manufacturing an apparatus of the above defined kind. Said method is characterized in that said bundle of semipermeable hollow fibers is arranged in said cylindrical open ended main part of the housing, supported by a ring having a low adhesive capacity as regards the material of the end walls, said ring being extended outside the open ends of the hollow fibers serving as an inlet and/or outlet for a flushing fluid for the flushing of the inside and the outside of the fibers and in that said ring thereafter is used as a part of a mould for moulding said end walls, whereafter it is cut together with the fibers in order to open the fiber ends, whereupon the apparatus finally is provided with said end caps.

## BRIEF DESCRIPTION OF DRAWINGS

Fig 1-3 show schematically one end of a first embodiment of the present invention at three different phases of its manufacturing.

Fig 4-6 show in the same way a second embodiment of the present invention.

Fig 7-9 and fig 9a show in the same way a third embodiment of the present invention.

## BEST MODES OF CARRYING OUT THE INVENTION

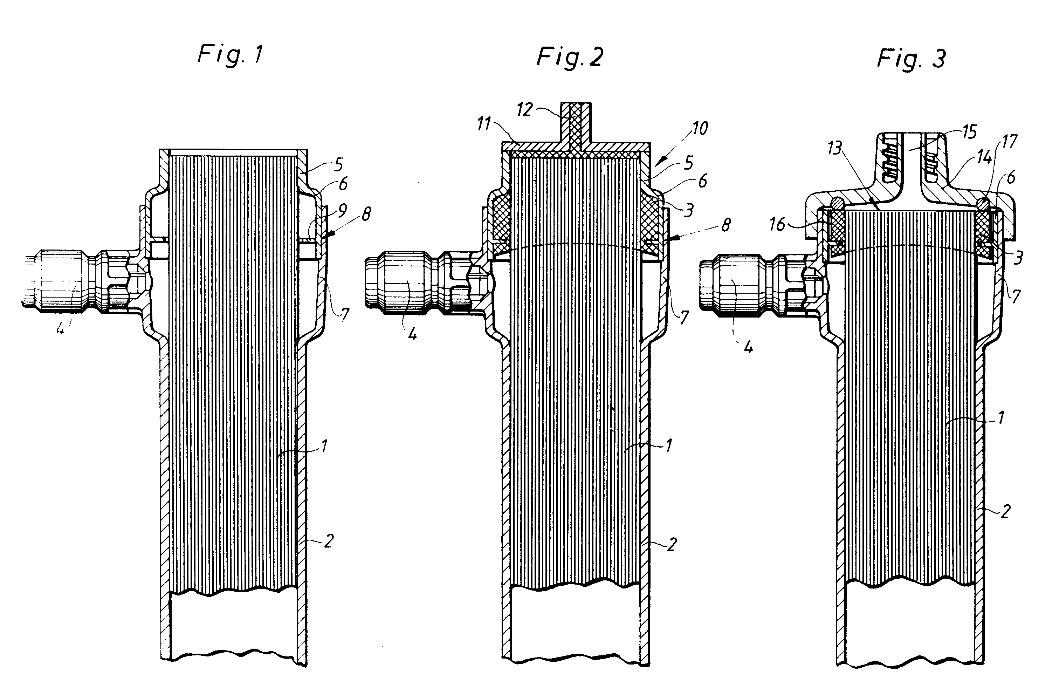

Fig 1-3 are showing one end of an apparatus according to the present invention during three different phases of its manufacturing.

In fig 1 a bundle of hollow fibers 1 is shown in a housing 2 before the casting of the end walls 3, shown in fig 2 and 3. The housing is provided with an inlet and/or outlet 4 intended for a first fluid. A similar inlet and/or outlet may be arranged at the other end of the device (not shown). The bundle of hollow fibers 1 is supported by a restricted part 5 of a ring 6 arranged in an expanded part 7 of the housing 2. The ring 6 is arranged in a groove 8 in the expanded part 7. At its inside the ring 6 is provided with an inwardly extending flange 9 which is shown to reach to a point near the bundle of hollow fibers. Alternatively, it may reach up to the outermost hollow fibers in order to provide a secondary support for the bundle.

The ring 6 has as mentioned above a low adhesive capacity as regards the material of the end walls 3. It may for instance be made of polypropylene when the end walls are made of polyurethane which is a material commonly used for said end walls. Alternatively, the ring 6 may be made of polyethylene, preferably LDPE, a material which is easier to cut, or Teflon (polytetrafluoroethylene), a

material having a very low adhesive capacity as regards most other materials.

In fig 2 a mould 10 has been provided in that the ring 6 has been completed with a lid 11 having an inlet 12 for the end wall material.

In fig 3 an end surface 13 has been provided by a cutting through the bundle of hollow fibers 1, the end wall 3, the ring 6, and the expanded part 7 of the housing 2. The apparatus has thereafter been provided with a lid 14 with an inlet and/or outlet 15 intended for a second fluid which may be fed through the fibers to a similar inlet and/or outlet 15 arranged at the opposite end of the apparatus (not shown).

Due to the relationship between the material of the end wall 3 and the ring 6 a small gap 16 has been provided between those two details. A sealing ring 17 has therefore been provided between the lid 14 and the end surface 13 in order to provide an effective seal between the two fluids involved, i e the fluids inside and outside the hollow fibers. It is to be observed that the gap 16 is not necessary. In practice it may be enough with a reduction of the attachment between the endwall 3 and the ring 6. In order to prevent leakage the lid 14 is preferably tightly welded to the outer surface of the expanded part 7 of the housing. Other types of sealings are, however, possible.

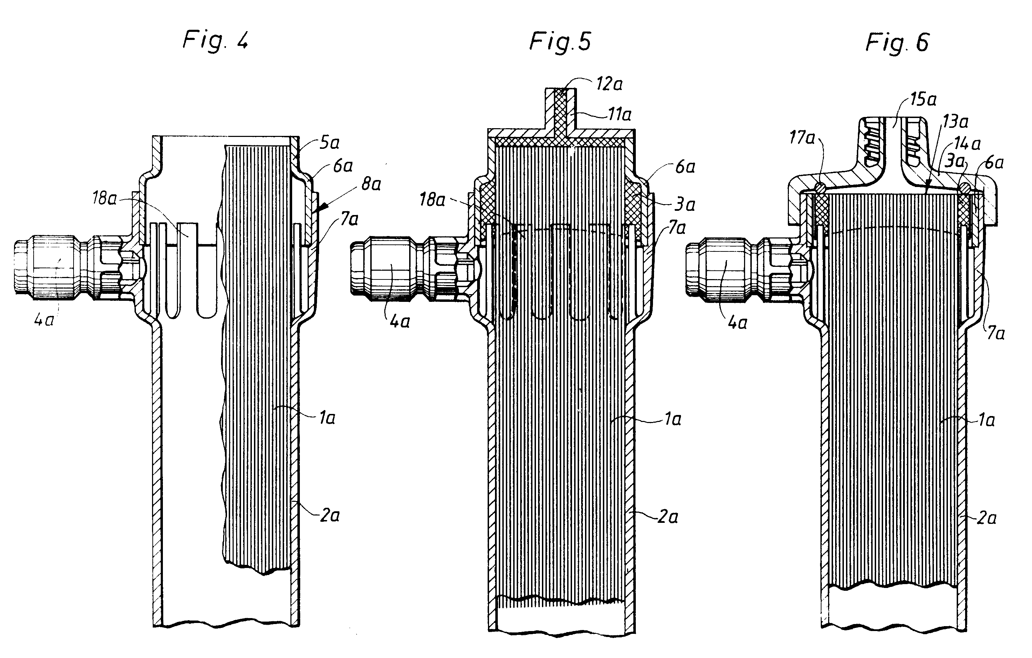

Fig 4-6 are showing a second embodiment of the apparatus according to the present invention. Many details are identical or similar to those shown in fig 1-3 and have therefore been given the same reference numerals, but with the addition of a.

Consequently, the apparatus shown is built up of a bundle of hollow fibers 1a in a housing 2a having an expanded part 7a. In a groove 8a in said expanded part 7a a slightly modified ring 6a is inserted. This ring 6a is in the same way as the ring 6 provided with a restricted part 5a supporting the bundle of hollow fibers 1a. An inlet 4a corresponds to the inlet 4.

The main difference between the embodiment according to fig 4-6 and the above described embodiment is a number of fingers or tongues 18a arranged in the expanded part 7a of the housing 2a. Said fingers or tongues 18a are made as integral extensions of the inner wall of the housing 2a.

In fig 5 the ring 6a has been completed with a lid 11a with an opening 12a for the casting material for the end walls 3a. The fingers or tongues 18a are, as can be seen in this figure, partly embedded in said end walls 3a, providing a support for said end walls 3a in the longitudinal direction of the housing 2a.

In fig 6 the bundle of hollow fibers 1a, the end wall 3a, the ring 6a and the expanded part 7a of the housing 2a have been cut, providing an end surface 13a. The apparatus has furthermore been provided with a lid 14a with an inlet and/or outlet 15a and with a sealing ring 17a.

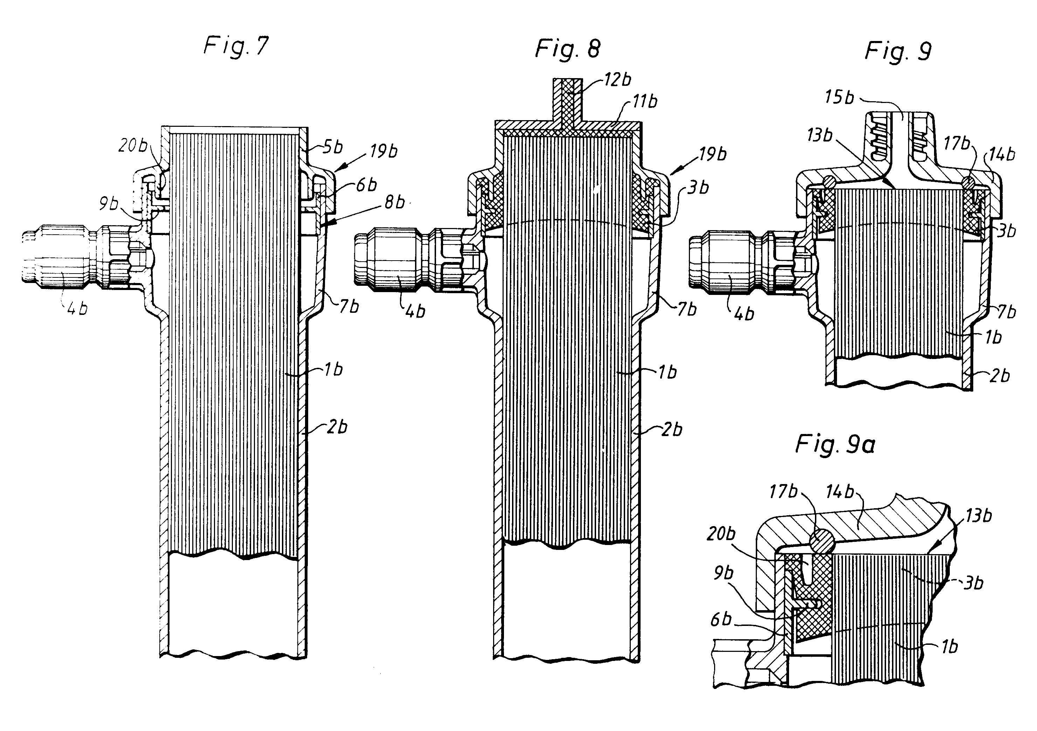

Fig 7-9 and 9a are showing a preferred third embodiment of the apparatus according to the present invention. Many of the details shown are identical to or similar to the details of fig 1-6 and have therefore been given the same reference numerals, but with the addition of b.

Consequently, a bundle of hollow fibers 1b is arranged in a housing 2b provided with an expanded part 7b with an inlet and/or outlet 4b. A first ring 6b similar to the ring 6 is arranged in a groove 8b in the expanded part 7b of the housing 2b. The ring 6b is provided with an inner flange 9b.

The main difference between the embodiment described in the fig 7-9 and 9a and the embodiment shown in fig 1-3 is a second ring 19b arranged partly outside the expanded part 7b of the housing 2b. Said second ring 19b is provided on one hand with a restricted part 5b corresponding to the part 5 of the ring 6 and on the other hand with an inner longitudinal extending flange 20b intended to be partly embedded in the end wall 3b.

In fig 8 the ring 19b has been provided with a lid 11b with an opening 12b for the feeding of the casting material for the end walls 3b.

In fig 9 and the enlargement shown in fig 9a a cut has been made through the expanded part 7b of the housing 2b, the end wall 3b, the flange 20b and the bundle of hollow fibers 1b, providing an end surface 13b. The apparatus has thereafter been provided with a lid 14b with an opening 15b and with a sealing ring 17b in the same way as the above described embodiments.

Due to the fact that the ring 6b does not extend up to the end surface 13b an attachment and therefore a sealing is provided between the material of the end wall 3b and the inside of the expanded part 7b of the housing 2b. The lid 14b does therefore not have to be tightly attached to the outside wall of the expanded part 7b of the housing 2b. It may for instance be attached to the housing 2b by means of a thread arranged between the lid 14b and the expanded part 7b.

PREFERRED MODE OF THE METHOD ACCORDING TO THE INVENTION

The present invention relates also to a method of manufacturing a diffusion and/or filtration apparatus of the kind described in for instance fig 1-3, i e including a housing 2 consisting of a cylindrical open ended main part closed by two endcaps 14 and being provided with an inlet 15 and an outlet (not shown) for a first fluid and at least one outlet 4 for a second fluid, said first fluid being adapted to flow through the fibers 1 of a bundle of semipermeable hollow fibers arranged between two end walls 3 within the housing 2 and said second fluid being adapted to be removed from the space outside the fibers 1 through said at least one outlet 4 for the second fluid, the ends of the hollow fibers 1 being moulded into said end walls 3 and opened by a cutting.

The method according to the invention is characterized in that said bundle of semipermeable hollow fibers 1 is arranged in said cylindrical open ended main part of the housing 2, supported by a ring 6 having a low adhesive capacity as regards the material of the end walls 3, said ring 6 being extended outside the open ends of the hollow fibers 1, serving as an inlet and/or outlet for a flushing fluid for the flushing of the inside and the outside of the fibers 1 and in that said ring 6 thereafter is used as a part of a mould for moulding said end walls 3 whereafter it is cut together with the fibers 1 in order to open the fiber ends, whereupon the apparatus finally is provided with said end caps 14.

All the above described figures show one end of an apparatus according to the invention. Normally the opposite end is made identical with the end shown in the drawings. This is true when for instance the apparatus is intended to be used for dialysis, a treatment which needs as well an inlet as an outlet for the dialysis liquid. If, however, the apparatus is intended to be used for filtration only it is enough to have one outlet 4 only for the filtrate.

The invention is of course not restricted to only the embodiments and the method described above, but may be varied within the scope of the following claims.

## Claims

1.  A diffusion and/or filtration apparatus, including a housing (2) consisting of a cylindrical open-ended main part closed by two end caps (14) and being provided with an inlet (15) and an outlet for a first fluid and at least one outlet (4) for a second fluid, said first fluid being adapted to flow through the fibers (1) of a bundle of semipermeable hollow fibers arranged beteen two end walls (3) within the housing (2) and said second fluid being adapted to be removed from the space outside the fibers (1) through said at least one outlet (4) for the second fluid, the ends of the hollow fibers (1) being moulded into said end walls (3) and opened by a cutting, **characterized** in that the attachment between the moulded end walls (3) and the housing (2) is reduced or eliminated by providing therebetween a ring (6) having a low adhesive capacity as regards the material of the end walls (3) and in that said end walls (3) are supported so that they are prevented from moving in the longitudinal direction of the housing (2).

2. Apparatus according to claim 1, **characterized** in that said support of the end walls (3) is provided by a flange (9) arranged on the ring (6).

3. Apparatus according to claim 2**, characterized** in that ring (6) is supported in the longitudinal direction by the housing (2).

4. Apparatus according to claim 3, **characterized** in that the ring (6) is arranged in a groove (8) in the inside wall of the housing (2).

5. Apparatus according to any of the preceding claims, **characterized** in that the ring (6) has an inner flange (9) providing a support for the bundle of hollow fibers (1) especially before the moulding of the end walls.

6. Apparatus according to any of the preceding claims, **characterized** by a second ring (19b) arranged at the outer end surfaces (13b) of the end walls (3b) providing a groove in each of said end surfaces, said ring having also a low adhesive capacity as regards the material of the end walls (3b).

7. Apparatus according to claim 1, **characterized** in that said support is provided by the housing (2a).

8. Apparatus according to claim 7, **characterized** in that the end walls (3a) are provided with two expanded parts (7a) of the housing (2a) and in that said support is provided by tongues (18a) which are made integral with the housing (2a) and partly moulded into the end walls (3a).

9. Apparatus according to any of the preceding claims, **characterized** in that the two end caps (14) are tightly attached to the housing (2), preferably by welding or glueing.

10. A method of manufacturing a diffusion and/or filtration apparatus including a housing (2) consisting of a cylindrical open-ended main part closed by two end caps (14) and being provided with an inlet (15) and an outlet for a first fluid and at least one outlet (4) for a second fluid, said first fluid being adapted to flow through the fibers (1) of a bundle of semipermeable hollow fibers (1) arranged between two end walls (3) within the housing (2) and said second fluid being adapted to be removed from the space outside the fibers (1) through said at least one outlet (4) for the second fluid, the ends of the hollow fibers (1) being moulded into said end walls (3) and opended by a cutting, **characterized** in that said bundle of semipermeable hollow fibers (1) is arranged in said cylindrical open ended main part of the housing (2), supported by a ring (6) having a low adhesive capacity as regards the material of the end walls (3), said ring (6) being extended outside the open ends of the hollow fibers (1) serving as an inlet and/or outlet for a flushing fluid for the flushing of the inside and the outside of the fibers (1) and in that said ring (6) thereafter is used as a part of a mould for moulding said end walls (3), whereafter it is cut together with the fibers (1) in order to open the fiber ends, whereupon the apparatus finally is provided with said end caps (14).

**Revendications**

1. Appareil de diffusion et/ou de filtration, incluant un boîtier (2) constitué par une partie principale cylindrique ouverte à ses extrémités qui est fermée par deux couvercles d'extrémité (14) et qui est munie d'une entrée (15) et d'une sortie pour un premier fluide ainsi que d'au moins une sortie (4) pour un second fluide, ledit premier fluide étant adapté pour s'écouler au travers des fibres (1) d'un faisceau de fibres creuses semi-perméables agencé entre deux parois d'extrémité (3) à l'intérieur du boîtier (2) et ledit second fluide étant adapté pour être extrait de l'espace externe aux fibres (1) au travers d'au moins une sortie (4) pour le second fluide, les extrémités des fibres creuses (1) étant moulées à l'intérieur desdites parois d'extrémité (3) et étant ouvertes par une découpe, caractérisé en ce que la fixation entre les parois d'extrémité moulées (3) et le boîtier (2) est réduite ou éliminée en disposant entre eux une bague (6) ayant une capacité d'adhérence faible par comparaison avec celle du matériau des parois d'extrémité (3) et en ce que lesdites parois d'extrémité (3) sont supportées de telle sorte qu'elles soient empêchées de se déplacer suivant la direction longitudinale du boîtier (2).

2. Appareil selon la revendication 1, caractérisé en ce que ledit support des parois d'extrémité (3) est constitué par une collerette (9) agencée sur la bague (6).

3. Appareil selon la revendication 2, caractérisé en ce que la bague (6) est supportée suivant la direction longitudinale par le boîtier (2).

4.  Appareil selon la revendication 3, caractérisé en ce que la bague (6) est agencée dans une gorge (8) ménagée dans la paroi interne du boîtier (2).

5.  Appareil selon l'une quelconque des revendications précédentes, caractérisé en ce que la bague (6) a une collerette interne (9) qui constitue un support pour le faisceau de fibres creuses (1), tout particulièrement avant le moulage des parois d'extrémité.

6.  Appareil selon l'une quelconque des revendications précédentes, caractérisé par une seconde bague (19b) agencée au niveau des surfaces d'extrémité externes (13b) des parois d'extrémité (3b) en ménageant une gorge dans chacune desdites surfaces d'extrémité, ladite bague ayant également une capacité d'adhérence faible par comparaison avec celle du matériau des parois d'extrémité (3b).

7.  Appareil selon la revendication 1, caractérisé en ce que ledit support est constitué par le boîtier (2a).

8.  Appareil selon la revendication 7, caractérisé en ce que les parois d'extrémité (3a) sont munies de deux parties élargies (7a) du boîtier (2a) et en ce que ledit support est muni de languettes (18a) qui font corps avec le boîtier (2a) et qui sont partiellement moulées à l'intérieur des parois d'extrémité (3a).

9.  Appareil selon l'une quelconque des revendications précédentes, caractérisé en ce que les deux couvercles d'extrémité (14) sont fixés hermétiquement au boîtier (2), de préférence par soudage ou collage.

10. Procédé de fabrication d'un appareil de diffusion et/ou de filtration incluant un boîtier (2) constitué par une partie principale cylindrique ouverte à ses extrémités qui est fermée par deux couvercles d'extrémité (14) et qui est munie d'une entrée (15) et d'une sortie pour un premier fluide ainsi que d'au moins une sortie (4) pour un second fluide, ledit premier fluide étant adapté pour circuler au travers des fibres (1) d'un faisceau de fibres creuses semi-perméables agencé entre deux parois d'extrémité (3) à l'intérieur du boîtier (2) et ledit second fluide étant adapté pour être extrait de l'espace externe aux fibres (1) au travers d'au moins une sortie (4) pour le second fluide, les extrémités des fibres creuses (1) étant moulées à l'intérieur desdites parois d'extrémité (3) et étant ouvertes par une découpe, caractérisé en ce que ledit faisceau de fibres creuses semi-perméables (1) est agencé dans ladite partie principale cylindrique ouverte à ses extrémités du boîtier (2) et est supporté par une bague (6) qui a une capacité d'adhérence faible par comparaison avec celle du matériau des parois d'extrémité (3), ladite bague (6) étant élargie à l'extérieur des extrémités ouvertes des fibres creuses (1) et servant d'entrée et/ou de sortie pour un fluide de rinçage destiné au rinçage de l'intérieur et de l'extérieur des fibres (1) et en ce que ladite bague (6) est ensuite utilisée en tant que partie d'un moule pour mouler lesdites parois d'extrémité (3), à la suite de quoi elle est coupée en même temps que les fibres (1) afin d'ouvrir les extrémités des fibres et de ce fait, l'appareil est finalement muni desdits couvercles d'extrémité (14).

**Patentansprüche**

1.  Diffusions-und/oder Filtriergerät, einschließlich eines Gehäuses (2), welches aus einem zylindrischen Hauptteil mit offenen Enden besteht, welche durch zwei Endkappen (14) verschlossen und mit einem Einlaß (15) und einem Auslaß für ein erstes Fluid sowie zumindest einen Auslaß (4) für ein zweites Fluid versehen werden, wobei das erste Fluid so ausgelegt ist, daß es durch die Fasern (1) eines Bündels von halbdurchlässigen hohlen Fasern fließt, welche zwischen den beiden Stirnwänden (3) innerhalb des Gehäuses (2) angeordnet sind, und wobei das zweite Fluid so ausgelegt ist, daß es aus dem Raum außerhalb der Fasern (1) durch den zumindest einen Auslaß (4) für das zweite Fluid entfernt werden kann, wobei die Enden der hohlen Fasern (1) in die Stirnwände (1) eingegossen und durch einen Schnitt geöffnet sind, dadurch gekennzeichnet, daß die Befestigung zwischen den gegossenen Stirnwänden (3) und dem Gehäuse (2) vermindert oder eliminiert wird, indem ein Ring (6) dazwischen vorgesehen wird, der eine geringe Haftfähigkeit bezüglich des Materials der Stirnwände (3) hat, und daß die Stirnwände (3) so gehaltert werden, daß ihre Bewegung in Längsrichtung des Gehäuses verhindert wird.

2.  Gerät nach Anspruch 1, dadurch gekennzeichnet, daß die Halterung der Stirnwände (3) durch einen Flansch (9) gewährleistet wird, der an dem Ring (6) angeordnet ist.

3.  Gerät nach Anspruch 2, dadurch gekennzeichnet, daß der Ring (6) in Längsrichtung durch das Gehäuse (2) gehaltert wird.

**4.** Gerät nach Anspruch 3, dadurch gekennzeichnet, daß der Ring (6) in einer Nut (8) in der Innenwand des Gehäuses (2) angeordnet ist.

**5.** Gerät nach einem der vorstehenden Ansprüche, dadurch gekennzeichnet, daß der Ring (6) einen inneren Flansch (9) hat, der eine Halterung für das Bündel hohler Fasern (1) insbesondere vor dem Gießen der Stirnwände bietet.

**6.** Gerät nach einem der vorstehenden Ansprüche, gekennzeichnet durch einen zweiten Ring (19b), der an den äußeren Endflächen (13b) der Stirnwände (3b) angeordnet ist, unter Vorsehen einer Nut in jeder der Endflächen, wobei der Ring ebenfalls eine geringe Haftfähigkeit bezüglich des Materials der Stirnwände (3b) hat.

**7.** Gerät nach Anspruch 1, dadurch gekennzeichnet, daß die Halterung von dem Gehäuse (2a) bereitgestellt wird.

**8.** Gerät nach Anspruch 7, dadurch gekennzeichnet, daß die Stirnwände (3a) mit bzw. in zwei aufgeweiteten Teilen (7a) des Gehäuses (2a) versehen sind und daß die Halterung durch Zungen (18a) bereitgestellt wird, welche einstückig mit dem Gehäuse (2a) ausgebildet und teilweise in die Stirnwände (3a) eingegossen sind.

**9.** Gerät nach einem der vorstehenden Ansprüche, dadurch gekennzeichnet, daß die beiden Endkappen (14) fest an dem Gehäuse (2) angebracht sind, vorzugsweise durch Schweißen oder Kleben.

**10.** Verfahren zum Herstellen eines Diffusions- und/oder Filtriergerätes einschließlich eines Gehäuses (2), welches aus einem zylindrischen Hauptteil mit offenen Enden besteht, welche durch zwei Endkappen (14) verschlossen werden und mit einem Einlaß (15) und einem Auslaß für ein erstes Fluid sowie zumindest einen Auslaß (4) für ein zweites Fluid versehen sind, wobei das erste Fluid so ausgelegt ist, daß es durch die Fasern (1) eines Bündels von halbdurchlässigen hohlen Fasern (1) fließt, welche zwischen zwei Stirnwänden (3) innerhalb des Gehäuses (2) angeordnet sind, und das zweite Fluid so ausgelegt ist, daß es aus dem Raum zwischen den Fasern (1) durch den zumindest einen Auslaß für das zweite Fluid entfernt werden kann, wobei die Enden der hohlen Fasern (1) in die Stirnwände (3) eingegossen und durch einen Schnitt geöffnet sind, dadurch gekennzeichnet, daß das Bündel halbdurchlässiger hohler Fasern (1) in dem zylindrischen Hauptteil des Gehäuses (2) mit offenen Enden angeordnet ist, von einem Ring (6) gehalten wird, welcher eine geringe Haftfähigkeit bezüglich des Materials der Stirnwände (3) hat, wobei der Ring (6) sich nach außerhalb der offenen Enden der hohlen Fasern (1) erstreckt und als Einlaß und/oder Auslaß für ein Spülfluid zum Spülen der Innenseite und der Außenseite der Fasern (1) dient, und daß der Ring (6) danach als Teil einer Gießform für das Gießen der Stirnwände (3) verwendet wird, wonach er zusammen mit den Fasern (1) durchschnitten wird, um die Faserenden zu öffnen, woraufhin das Gerät schließlich mit den Endkappen (14) versehen wird.

Fig. 1

Fig. 2

Fig. 3

*Fig. 4*

*Fig.5*

*Fig. 6*

EP 0 305 687 B1

Fig. 7

Fig. 8

Fig. 9

Fig. 9a

EP 0 305 687 B1